# FASCICULE DE BREVET EUROPEEN

(11) **EP 3 580 272 B1**
(45) Date de publication et mention de la délivrance du brevet: **07.10.2026**
(21) Numéro de dépôt: 18706811.9
(22) Date de dépôt: 09.02.2018
(51) Int. Cl.: C08L 5/08, C08L 1/28, A61K 31/728, A61K 31/717

(54) **SOLUTION VISCOÉLASTIQUE AQUEUSE ET SON UTILISATION DANS UN DISPOSITIF D'INJECTION**
WÄSSRIGE VISKOELASTISCHE LÖSUNG UND VERWENDUNG DAVON IN EINER INJEKTIONSVORRICHTUNG
AQUEOUS VISCOELASTIC SOLUTION AND USE THEREOF IN AN INJECTION DEVICE

(30) Priorité: 10.02.2017 FR 1751134
(43) Date de publication de la demande: 18.12.2019
(73) Titulaire: POLYMEREXPERT, 33600 Pessac (FR)
(72) Inventeur: DOLATKHANI, Marc, 33610 Cestas (FR); PAGNOUX, Anne, 33114 Le Barp (FR); DAKHLI, Sonia, 33140 Villenave D'Ornon (FR)
(74) Mandataire: Santarelli
(86) Numéro de dépôt international: PCT/FR2018/050326
(87) Numéro de publication internationale: WO 2018/146429

(56) Documents cités:
- EP-A1- 1 992 329
- WO-A1-2010/118080
- WO-A1-2013/117863
- WO-A1-94/01468
- US-A- 4 500 676
- US-A1- 2009 060 973
- US-A1- 2009 118 761
- US-B1- 7 345 117

## Description

L'invention concerne une solution viscoélastique aqueuse comprenant :
- au moins un polymère choisi parmi l'acide hyaluronique et ses dérivés, tels que l'un de ses sels, et les dérivés cellulosiques et leurs sels, en particulier l'hydroxypropylméthylcellulose et ses sels, et
- au moins un polymère hydrosoluble de type polyéther-polyuréthane ou polyéther-polyester-uréthane,
ainsi que son utilisation dans un dispositif d'injection, en particulier dans un dispositif destiné à l'injection d'un implant intraoculaire.

L'invention concerne également un dispositif destiné à l'injection d'un implant intraoculaire, comprenant un corps d'injection, une cartouche de chargement et un piston d'injection, dans lequel la cartouche de chargement contient ladite solution viscoélastique aqueuse.

Le remplacement du cristallin de l'œil affecté par la cataracte est assuré par des implants intraoculaires. La chirurgie utilisant la phacoémulsification permet la destruction du cristallin naturel et son élimination par une petite incision. Il a été développé des implants en matériaux souples et pliables pouvant être insérés à l'aide de dispositifs d'injection à travers une micro-incision.

Le système d'injection est composé d'un corps tubulaire dans lequel coulisse le piston d'injection surmonté d'une cartouche de chargement de l'implant ophtalmique et d'un embout de forme conique dont le diamètre diminue au fur et à mesure que l'on se rapproche de l'extrémité d'injection.

Il existe deux types de dispositifs d'injection utilisés en chirurgie opthalmique, communément appelés « injecteurs ophtalmiques » :
- injecteur monobloc pour lequel la cartouche de chargement de l'implant et l'embout sont liés au corps tubulaire de l'injecteur.
- injecteur constitué de deux pièces et pour lequel la cartouche de chargement de l'implant et l'embout sont séparés du corps tubulaire. Les deux pièces s'emboîtent après le chargement de l'implant dans la cartouche (par exemple, le système Viscojet^{®} commercialisé par Medicel AG).

Quel que soit le type d'injecteur retenu, le chirurgien appuie sur le piston dont l'extrémité pousse l'implant qui se trouve dans la cartouche ; ce dernier est contraint de plus en plus dans l'embout de l'injecteur et finit par ressortir de l'injecteur totalement plié. Il est ainsi possible d'injecter un implant de plus de 6 mm de diamètre par une incision de taille inférieure à 3 mm. De très grandes contraintes sont alors exercées sur l'implant lors du transfert. Pour limiter la force d'injection et permettre à l'implant de sortir de l'embout sans dommages, il est nécessaire, d'une part, d'optimiser la géométrie de l'injecteur et, d'autre part, d'utiliser un « lubrifiant ».

Le choix du matériau constitutif de l'embout et de la cartouche, ainsi que l'optimisation de sa géométrie, ne suffisent cependant pas pour injecter des implants via de micro-incisions de façon satisfaisante. Il est impératif d'utiliser un lubrifiant permettant à l'implant de glisser dans l'embout et la cartouche. Les approches permettant la lubrification décrites dans la littérature sont l'utilisation d'un agent de migration et la mise en œuvre d'un revêtement.

La première voie concerne l'utilisation d'un agent de migration (en anglais « blooming agent ») intégré par mélange (ou, selon le terme usuel, « compoundage ») dans le polymère thermoplastique constituant l'embout et la cartouche. Il s'agit généralement d'une molécule organique oléophile ou tensioactive de faible masse molaire, contenant 10 à 30 atomes de carbone par molécule, dérivée d'acide carboxylique et de type monostéarate de glycérol (GMS), monopalmitate de glycérol ou bien monooléate de glycérol etc... Mise en mélange avec le polypropylène ou bien le polyamide, ce type de molécule se répartit uniformément dans le support thermoplastique juste après injection et finit par migrer à la surface du support après plusieurs jours voire plusieurs semaines. Ce phénomène de migration est lié à la petite taille de la molécule tensioactive qui est mobile comparée aux chaînes macromoléculaires du matériau thermoplastique.

WO2013/117863 divulgue l'utilisation séparée de l'acide hyaluronique ou l'hydroxypropylméthylcellulose (en solution aqueuse) et du polyéther-polyuréthane dans des solutions d'injection ophtalmique.

A titre d'exemple, les brevets US 6 733 507, US 6 679 891 et US 6 083 230 décrivent des cartouches en polypropylène (PP) contenant un agent lubrifiant qui migre à la surface par phénomène de migration (« blooming »). Cette approche présente deux inconvénients majeurs.

Le premier est la présence de traces blanches sur les implants injectés. Elles sont dues au fait que l'agent de migration n'est pas lié à la surface de l'embout et est entraîné lors de l'injection. En effet, les agents de migration utilisés ne sont pas hydrosolubles et leur élimination n'est possible qu'après de nombreux rinçages, une fois l'implant injecté.

Le second inconvénient est lié à la cinétique de migration de l'agent de migration à la surface du support. Cette migration peut durer plusieurs jours, voire plusieurs semaines en fonction des conditions de mise en œuvre (injection-moulage de la pièce), de la température de stockage, de post-traitement, des conditions de stérilisation, etc..., avant qu'une quantité suffisante de lubrifiant se trouve à la surface de la cartouche d'injection. La qualité de la lubrification va donc dépendre du temps d'attente entre la fabrication de l'injecteur et l'utilisation de celui-ci par le praticien.

La non-reproductibilité de la lubrification et la présence de tâches blanchâtres sur les implants lors de l'injection représentent les principaux défauts de l'utilisation de ces agents de migration.

Une deuxième approche (approche « revêtement », en anglais « coating ») permettant le glissement des implants dans les embouts/cartouches des injecteurs est la mise en place d'un revêtement hydrophile à l'intérieur de l'embout. Le principe de lubrification consiste à gonfler le revêtement hydrophile par l'addition d'un produit visqueux (solution d'hyaluronate ou bien d'hydroxypropylméthyl cellulose) et à glisser ainsi sur un film d'eau formé à l'interface.

Les brevets ou demandes de brevet JP56090838, JP3254752, US 5 716 364, EP1949871, décrivent la possibilité de réaliser un revêtement permettant de diminuer ou éliminer la friction entre l'implant et la cartouche.

Le revêtement hydrophile est lié soit par des liaisons chimiques covalentes soit par des liaisons physiques à la surface de l'embout/cartouche.

En ce qui concerne l'approche « revêtement », les propriétés glissantes sont constantes au cours du temps et n'évoluent pas comme dans le cas des agents de migration. L'inconvénient majeur des procédés qui consistent à revêtir les dispositifs réside dans la complexité d'élaboration de tels revêtements, en particulier lorsqu'il s'agit de la surface interne des injecteurs. Cette approche nécessite d'opérer en plusieurs étapes : i) activation de la surface ii), dépôt du film polymère ou polymérisation/greffage puis iii) évaporation du solvant ou du composé non réagi, enfin iv) contrôle des caractéristiques du film (homogénéité/ épaisseur), etc. Ces procédures augmentent de manière significative le temps de production et le coût d'accès à des dispositifs possédant une surface glissante.

Quel que soit le type de glissant utilisé (agent de migration de type GMS ou revêtement) au niveau de la cartouche, l'injection de l'implant est réalisée après addition d'une solution viscoélastique (solution d'hyaluronate ou bien d'hydroxypropylméthyl cellulose).

On a maintenant trouvé que l'addition d'un agent glissant dans la solution viscoélastique permet une injection aisée de l'implant sans aucun traitement préalable de la cartouche de l'injecteur. Cette approche pallie les inconvénients des approches existantes sur le marché, à savoir la non-reproductibilité et la présence de traces sur les cartouches compoundées avec des agents de migration de type GMS d'une part, et, d'autre part le coût élevé des revêtements élaborés à la surface des cartouches.

Il y a un grand intérêt à disposer de solutions viscoélastiques permettant, d'une part, le glissement de l'implant dans l'injecteur, et d'autre part, apte à être extraite aisément en fin de procédure.

Selon les données du PMSI 2009 (Programme de Médicalisation des Systèmes d'Information), l'extraction extracapsulaire du cristallin par phacoémulsification, avec implantation de cristallin artificiel (lentille intraoculaire) dans la chambre postérieure de l'œil est le motif de séjour hospitalier le plus fréquent en France (plus de 98 % des motifs de séjours).

Cette opération, largement menée pour répondre à des problèmes de cataracte, nécessite une incision oculaire qui entraîne une perte de l'humeur aqueuse du segment antérieur de l'œil. Pour rééquilibrer la pression de ce segment, une solution viscoélastique est injectée. Elle est caractérisée par une forte viscosité qui permet le maintien du volume de l'œil et facilite également les gestes chirurgicaux de découpe capsulaire antérieure (capsulorhexis). Ce fluide visqueux assure ainsi la protection de l'endothélium cornéen avant la phase de phacoémulsification (destruction du cristallin par ultrasons et aspiration des fragments), à l'issue de laquelle il sera évacué.

Durant l'implantation de la lentille, un fluide de plus basse viscosité est utilisé notamment dans le sac cristallin, et/ou dans le sulcus ciliaire afin de faciliter la mise en place de l'implant.

Plusieurs solutions viscoélastiques à usage chirurgical intraoculaire sont disponibles commercialement. Elles sont essentiellement composées de hyaluronate de sodium (NaHA), associée ou non à de la chondroïtine sulfate ou à un dérivé de cellulose tel que l'hydroxypropylméthylcellulose (HPMC). Une telle solution est décrite, par exemple, dans la demande US 2004/0241155.

Les solutions viscoélastiques à base d'hyaluronate de sodium se différencient par l'origine de l'acide hyaluronique, la masse moléculaire (entre 0,22 MDa et 6 MDa) et la viscosité au repos (entre 10 Pa.s et 7 000 Pa.s) (source : Les solutions viscoélastiques, CHIRURGIE DE LA CATARACTE, Cahier des dispositifs médicaux Le Moniteur Hospitalier n° 259 du 01/10/2013).

Il existe une autre catégorie de solution viscoélastique, à base d'hydroxylpropylméthylcellulose (HPMC) ou hypromellose, d'origine végétale. L'hypromellose est caractérisée par sa capacité d'étalement et donc sa capacité de protection de l'endothélium. Elle est de faible viscosité. Son extraction en fin de procédure est plus difficile.

La demande EP 1496037 écrit un produit de modification de l'acide hyaluronique, dans lequel l'acide hyaluronique ou un de ses sels pharmaceutiquement acceptables est lié à un polymère à bocs. Ce produit de modification est utilisable comme produit thérapeutique en chirurgie ophtalmique ou réparatrice, ou encore pour le traitement de maladies articulaires.

Les solutions viscoélastiques selon l'invention sont des solutions viscoélastiques à usage chirurgical intraoculaire dont la spécificité est de posséder des propriétés physicochimiques permettant à l'implant de glisser dans l'injecteur, c'est-à-dire une action mécanique et non thérapeutique. Cette propriété repose sur la composition de la solution dont l'ingrédient principal est un polymère choisi parmi l'acide hyaluronique et ses sels, le hyaluronate de sodium étant préféré, et les dérivés cellulosiques, en particulier l'hydroxypropylméthycellulose et ses sels, auquel est associé, de préférence en faible quantité, un polymère hydrosoluble de type polyéther-uréthane ou polyéther-polyester-uréthane, présentant des propriétés intrinsèques de lubrification en milieu aqueux.

Ledit polymère choisi parmi l'acide hyaluronique et ses sels, le hyaluronate de sodium étant préféré, et les dérivés cellulosiques et leurs sels (en particulier l'hydroxypropylméthycellulose et ses sels) est, de préférence, présent dans la solution viscoélastique aqueuse en quantité largement supérieure à celle du polymère hydrosoluble de type polyéther-polyuréthane ou polyéther-polyester-uréthane.

Par « sel », on entend dans la présente description un sel pharmaceutiquement acceptable.

Le comportement des solutions viscoélastiques hautement pseudoplastiques, c'est-à-dire dont la viscosité diminue sous cisaillement, utilisées selon l'invention permet à la fois une mise en place facilitée de l'implant et un retrait aisé de la solution visqueuse lors de l'intervention chirurgicale, ce qui leur confère un grand avantage par rapport aux solutions viscoélastiques existantes. Cet avantage est apporté par la propriété de glissance conférée par le polymère hydrosoluble contenu dans la solution. La glissance peut être quantifiée par la mesure de la force nécessaire à l'injection de lentille intraoculaire.

Les solutions viscoélastiques selon l'invention sont des solutions stériles, non immunogènes, non toxiques, proches du pH physiologique de l'œil (6,8-7,6), transparentes, faciles à injecter et à retirer.

Selon la masse molaire du polymère choisi parmi l'acide hyaluronique et ses sels, et les dérivés cellulosiques, en particulier l'hydroxypropylméthycellulose et ses sels, les solutions viscoélastiques seront soit de type cohésif, soit de type dispersif, pour une utilisation séquentielle ou conjointe.

A titre d'exemple, les solutions viscoélastiques de type cohésif peuvent comprendre de l'acide hyaluronique de masse molaire comprise entre 1,5 MDa et 5MDa. On entend par « masse molaire » la masse molaire moyenne en masse (Mw). Alternativement, les solutions viscoélastiques de type dispersif peuvent contenir de l'acide hyaluronique de plus faible masse molaire, comprise entre 0,5 MDa et 1,5 MDa. Les solutions viscoélastiques peuvent, par exemple, contenir jusqu'à 0, 5%, notamment 0,25% (exprimé en poids/volume) de polymère glissant (polymère hydrosoluble de type polyéther-uréthane ou polyéther-polyester-uréthane) et sont constituées de solutions isotoniques garantissant un pH physiologique et une osmolalité de 300 à 350 mOsm/kg.

L'invention a donc pour objet une solution viscoélastique aqueuse comprenant :
- au moins un polymère choisi parmi l'acide hyaluronique et ses sels, et l'hydroxypropylméthylcellulose ou un de ses sels, et
- au moins un polymère hydrosoluble de type polyéther-polyuréthane ou polyéther-polyester-uréthane, ledit polymère hydrosoluble de type polyéther-polyuréthane ou polyéther-polyester-uréthane ne comportant pas de chaine grasse terminale ou pendante comportant au moins 10 atomes de carbone,
ladite solution viscoélastique ayant une viscosité comprise entre 10 et 7000 Pa.s.

Avantageusement, la solution viscoélastique aqueuse selon l'invention se présente sous forme de gel et aune viscosité comprise entre 10 et 7000 Pa.s, notamment entre 30 et 1000 Pa.s, plus particulièrement entre 50 et 200 Pa.s.

On notera que, dans la présente description, et sauf stipulation contraire, les gammes de valeur indiquées s'entendent bornes incluses.

La viscosité peut être mesurée selon les méthodes usuelles dans le domaine, par exemple par mesure de rhéologie en écoulement à l'aide d'un rhéomètre (par exemple, de marque TA et de type AR 1000). La courbe de viscosité en fonction du cisaillement permet d'obtenir la valeur de la viscosité à cisaillement nul.

De préférence, l'acide hyaluronique peut avoir une masse molaire moyenne en masse comprise entre 1,5 MDa et 5MDa, ou alternativement, comprise entre 0,5 MDa et 1,5 MDa.

De préférence, l'acide hyaluronique est sous forme de sel de préférence d'hyaluronate de sodium ou de potassium.

Un dérivé cellulosique préféré aux fins de l'invention est l'hydroxypropylméthylcellulose ou un de ses sels, notamment le sel de sodium ou de potassium.

De préférence, la teneur en polymère choisi parmi l'acide hyaluronique et/ou l'un de ses sels et les dérivés cellulosique, notamment l'hydroxypropylméthylcellulose, et/ou l'un de leurs sels, dans la solution viscoélastique aqueuse peut être comprise entre 0,5 et 3 %, de préférence entre 1 et 2,5 %, exprimée en poids/volume ou, autrement dit, entre 5 mg/mL et 30 mg/mL.

De préférence, l'acide hyaluronique et/ou l'un de ses sels, d'une part, et le dérivé cellulosique, notamment l'hydroxypropylméthylcellulose et/ou l'un de ses sels, d'autre part, sont présents dans ladite solution viscoélastique à raison de 5 à 25 mg/mL, en particulier de 10 à 25 mg/mL.

Des polymères thermogélifiants hydrosoluble de type polyéther-polyuréthane particulièrement adaptés l'utilisation selon l'invention sont les poly(urée-uréthane)s comportant des groupements poly(oxyde d'éthylène-b-oxyde de propylène-b-oxyde d'éthylène) (POE-b-POP-b-POE), en particulier des copolymères à blocs d'oxyde d'éthylène et d'oxyde de propylène ramifié comprenant des ponts uréthane, allophanate et urée tels que ceux décrits dans la demande WO0306536.

Ces poly(urée-uréthane)s sont obtenus par polycondensation de diisocyanates et de diols triblocs POE-b-POP-b-POE thermosensibles et peuvent comprendre des groupements uréthane et/ou allophanate, urée et/ou biuret. Ces polymères sont connus, notamment, sous le nom d'ExpertGel^{®} et commercialisés par la société PolymerExpert.

Ces polymères thermogélifiants hydrosoluble de type polyéther-polyuréthane ont, de préférence, une masse molaire moyenne en masse supérieure ou égale à 20 KDa, et, plus particulièrement, une masse molaire comprise entre 20 et 100 KDa.

Le polymère hydrosoluble de type polyéther-polyester-uréthane peut être un copolymère de polyéther et de polyester ramifié comprenant des ponts uréthane, allophanate et urée. Ces copolymères ont de préférence, une masse molaire moyenne en masse supérieure ou égale à 20 KDa, et, en particulier, comprise entre 20 et 100 KDa. Ces polyéther-polyester-uréthanes sont obtenus par polycondensation de diisocyanates et de diols polyéther et polyester et peuvent comprendre des groupements uréthane et/ou allophanate, urée et/ou biuret.

La teneur en polymère hydrosoluble de type polyéther-polyuréthane ou polyéther-polyester-uréthane dans la solution viscoélastique aqueuse peut être comprise entre 0,10 et 0,5 %, de préférence entre 0,10 et 0,25 %, exprimée en poids/volume ou, autrement dit, entre 1 mg/mL et 5 mg/mL, de préférence entre 1 mg/mL et 2,5 mg/mL.

L'invention a également pour objet l'utilisation d'une solution viscoélastique aqueuse telle que décrite plus haut, comprenant :
- au moins un polymère choisi parmi l'acide hyaluronique et ses sels, et les dérivés cellulosiques et leurs sels, et
- au moins un polymère hydrosoluble de type polyéther-polyuréthane ou polyéther-polyester-uréthane,
dans un dispositif d'injection.

L'invention a également pour objet une solution viscoélastique telle que décrite plus haut, pour son utilisation pour l'injection d'un implant intraoculaire hydrophile ou hydrophobe.

Tous les aspects généraux ou particuliers de ladite solution viscoélastique aqueuse, mentionnés dans la présente description, s'appliquent à ces utilisations.

Avantageusement, l'utilisation de ladite solution viscoélastique aqueuse permet de diminuer la force à exercer sur le piston dudit dispositif d'injection, communément appelée « force d'injection ».

Ledit dispositif d'injection peut être, selon un aspect préféré, un dispositif destiné à l'injection d'un implant intraoculaire (communément appelé « injecteur ophtalmique »).

Ladite solution viscoélastique joue le rôle d'agent glissant, sans pour autant revêtir les surfaces de la cartouche de l'injecteur.

Le dispositif destiné à l'injection d'un implant intraoculaire peut comprendre, en particulier, un corps d'injection, un embout, une cartouche de chargement et un piston d'injection.

Selon un aspect avantageux, la solution viscoélastique telle que décrite plus haut peut être introduite dans ladite cartouche de chargement et dans l'embout préalablement ou simultanément au chargement de l'implant intraoculaire. Avantageusement, ladite solution viscoélastique permet l'injection d'un implant intraoculaire hydrophile ou hydrophobe par un dispositif d'injection équipé d'un embout ayant une ouverture de diamètre égal ou inférieur à 2,6 mm avec une force d'injection inférieure à 30N, de préférence inférieure à 25N. Selon l'invention, ce résultat est obtenu alors même que la cartouche de chargement n'a subi aucun traitement de type « revêtement » et qu'aucun agent de migration n'a été intégré au polymère thermoplastique constituant l'embout.

L'invention concerne également un dispositif destiné à l'injection d'un implant intraoculaire, comprenant un corps d'injection, une cartouche de chargement et un piston d'injection, dans lequel la cartouche de chargement contient une solution viscoélastique telle que définie plus haut, dans tous ses aspects généraux et préférés.

L'invention est illustrée par les exemples ci-dessous. Les exemples 1 à 4 concernent la préparation de polymères hydrosolubles de type polyéther-uréthane et polyéther-polyester-uréthane utilisables aux fins de l'invention et les exemples 5 à 8 visent des solutions viscoélastiques selon l'invention et l'étude de leurs propriétés.

Les abréviations suivantes sont utilisées :
GMS : monostéarate de glycérol
NaHA : hyaluronate de sodium
HPMC : hydroxypropylméthylcellulose
PEG : polyéthylèneglycol

### Exemple 1 : préparation d'un polymère hydrosoluble de type polyéther-polyuréthane (EG230)

Le polymère EG230 est un polyuréthane composé d'un copolymère tri bloc oxyde ethylène - oxyde de propylène - oxyde d'éthylène reliés entre eux par des liaisons uréthane, allophanate et urée. Le polymère EG230 est obtenu par réaction entre les polymères présentant des groupes hydroxyles et un réactif isocyanate.

L'Adekanol F108 est séché dans un réacteur sous vide et sous agitation continue à 120°C de manière à ce que la totalité de l'eau soit retirée. Le PEG600 est placé dans un récipient permettant le chauffage et la mise sous vide et mis à fondre sous vide à 80°C jusqu'à fusion complète.

Après refroidissement du réacteur contenant le F108 à une température d'environ 70°C, on ajoute sous azote la 2-butanone préalablement séchée sur CaCl₂ et filtrée, puis l'eau, le diisodicyanate-4,4' de dicyclohexylméthane et le dibutyldilaurate d'étain (catalyseur). Les réactifs sont mélangés en maintenant le chauffage.

Lorsque la conversion des fonctions isocyanate atteint 58%, on ajoute en une seule fois le PEG600 sec et liquide (80°C), sous flux d'azote. Le chauffage est arrêté lorsqu'au moins 99% des isocyanates sont consommés.

Après arrêt du flux d'azote, on ajoute l'éthanol dans le milieu réactionnel sous agitation pendant 30 min. Le polymère EG230 est précipité dans 1,5 fois le volume de milieu réactionnel et séché à 40°C sous vide.

Sa composition est donnée dans le tableau 1 ci-dessous :

**Tableau 1**

| | Rapport Molaire | Masse molaire (g/mol) | Masse (g) |
|---|---|---|---|
| Adekanol F108 (commercialisé par ADEKA) | 1 | - | 300 |
| Diisocyanate-4,4' de dicyclohexylméthane | 2,06 | 262 | 15 |
| monométhyl ether du polyéthylène glycol (PEG 600) | 1,73 | 600 | 35 |
| 2-butanone sèche | - | | 900 mL |
| Eau déminéralisée | 0,2g/100g F108 | | 0,6 |
| Dibutyldilaurate d'étain | 500 ppm/F108 | | 0,15 |
| Ethanol 99% nature | 100mL/100 kg F108 | | 100 mL |

### Exemple 2 : préparation d'un polymère hydrosoluble de type polyéther-polyuréthane (EG68(0))

L'ExpertGel EG68(0) est un polyuréthane composé d'un copolymère tri bloc oxyde d'éthylène - oxyde de propylène - oxyde d'éthylène reliés entre eux par des liaisons uréthane, allophanate et urée. L'ExpertGel EG68(0) est obtenu par réaction entre les polymères représentant des groupes hydroxyles et un réactif isocyanate.

Brièvement, le Lutrol F68 (Kolliphor P188) est séché dans un réacteur sous vide et sous agitation continue à 120°C de manière à ce que la totalité de l'eau soit retirée. Le PEG600 est placé dans un récipient permettant le chauffage et la mise sous vide et mis à fondre sous vide à 80°C jusqu'à fusion complète.

Après refroidissement du réacteur contenant le F68 à une température d'environ 70°C, on ajoute sous azote la 2-butanone préalablement séchée sur CaCl₂ et filtrée, puis l'eau, le diisodicyanate-4,4' de dicyclohexylméthane et le dibutyldilaurate d'étain (catalyseur). Les réactifs sont mélangés en maintenant le chauffage.

Lorsque la conversion des fonctions isocyanate atteint 58%, on ajoute en une seule fois le PEG600 sec et liquide (80°C), sous flux d'azote. Le chauffage est arrêté lorsqu'au moins 99% des isocyanates sont consommés.

Après arrêt du flux d'azote, on ajoute l'éthanol dans le milieu réactionnel sous agitation pendant 30 min. Le polymère l'EG68(O) est précipité dans 1,5 fois le volume de milieu réactionnel et séché à 40°C sous vide.

Sa composition est donnée dans le tableau 2 ci-dessous :

**Tableau 2**

| | Rapport Molaire | Masse molaire (g/mol) | Masse (g) |
|---|---|---|---|
| Lutrol F68 (Kolliphor P188 commercialisé par BASF) | 1 | - | 300 ± 3 |
| Diisocyanate-4,4' de dicyclohexylméthane | 2,06 | 262 | 18,4 |
| monométhyl ether du polyéthylène glycol (PEG 600) | 1,82 | 600 | 37,1 |
| Dibutyldilaurate d'étain | 500 ppm/F68 | | 0,15 |
| 2-butanone sèche | - | | 0,9L |
| Eau déminéralisée | 0,2g/100g F68 | | 0,6 |
| Ethanol 99% nature | 5mL/kg F68 | | 0,15 |

### Exemple 3 : préparation d'un polymère hydrosoluble de type polyéther-polyuréthane (EG88(0))

Le polymère EG88(0) est un polyuréthane composé d'un copolymères tri bloc oxyde d'éthylène - oxyde de propylène - oxyde d'éthylène reliés entre eux par des liaisons uréthane, allophanate et urée. Le polymère EG88(0)est obtenu par réaction entre les polymères représentant des groupes hydroxyles et un réactif isocyanate. Le protocole de synthèse est similaire à celui du polymère EG68(0) de l'exemple 2, mais en remplaçant le Lutrol F68 par le Lutrol F88.

Sa composition est donnée dans le tableau 3 ci-dessous:

**Tableau 3**

| | Rapport Molaire | Masse molaire (g/mol) | Masse (kg) |
|---|---|---|---|
| Lutrol F88 (commercialisé par BASF) | 1 | - | 300 ± 3 |
| Diisocyanate-4,4' de dicyclohexylméthane | 2,06 | 262 | 14,2 |
| monométhyl ether du polyéthylène glycol (PEG 600) | 1,82 | 600 | 28,7 |
| Dibutyldilaurate d'étain | 500 ppm/F88 | | 0,15 |
| 2-butanone sèche | - | | 0,9 L |
| Eau déminéralisée | 0,2g/100g F88 | | 0,6 |
| Ethanol 99% nature | 5mL/kg F88 | | 0,15 |

### Exemple 4 : préparation d'un polymère hydrosoluble de type polyéther-polyester-uréthane (ES)

Le polymère ES est un copolymère de polyéther et polyester ramifié, présentant des ponts uréthane et allophanate (et très peu d'urée). Il est obtenu par réaction d'un polycaprolactone dihydroxyteléchélique et d'un polyéthylèneglycol et d'un diisocyanate.

Brièvement, la polycaprolactone (CAPA) et le PEG sont séchés dans un réacteur sous vide et sous agitation continue à 100°C. Lorsque la CAPA et le PEG sont totalement fondus, on maintient le chauffage et le vide de manière à ce que la totalité de l'eau contenue dans les polymères soit retirée.

Après refroidissement du réacteur à une température d'environ 80°C, on ajoute sous azote la 2-butanone préalablement séchée sur CaCl₂ et filtrée, puis le diisodicyanate-4,4' de dicyclohexylméthane et le carboxylate de bismuth (catalyseur). Les réactifs sont mélangés en maintenant le chauffage pendant au moins 15h à 60°C.

Après arrêt du flux d'azote, on ajoute l'éthanol dans le milieu réactionnel sous agitation pendant 30 min, puis on vérifie l'absence d'isocyanate. On dilue le milieu réactionnel avec 17L de 2-butanone, puis le polymère ES final est précipité dans l'heptane et séché à 40°C sous vide.

Sa composition est donnée dans le tableau 4 ci-dessous.

**Tableau 4**

| | Masse (g) |
|---|---|
| Polycaprolactone CAPA 2125 (commercialisé par Perstorp) | 305,5 |
| monométhyl ether du polyéthylène glycol (PEG 6000) | 6700 |
| Diisocyanate-4,4' de dicyclohexylméthane | 329,2 |
| Carboxylate de bismuth | 3,5 |
| 2-butanone sèche | 3,08 L |
| Ethanol 99% nature | 10mL |

### Exemple 5 : préparation d'une solution viscoélastique aqueuse comprenant un polymère hydrosoluble de type polyéther-polyuréthane ou polyéther-polyester-uréthane à 0,25% poids/volume

La première partie de la préparation est effectuée en salle propre. Une solution à 2,5% poids/volume de polymère hydrosoluble de type polyéther-uréthane tel que ceux indiqués dans les exemples 1 à 3 ou polyester-polyéther-uréthane tel que celui de l'exemple 4 est réalisée par dissolution sous agitation mécanique en introduisant le polymère (2,5 g) sous forme de poudre dans de l'eau isotonique tamponnée à pH 7 (97,5 mL). Après complète dissolution, la solution est stérilisée par filtration à 0,2 micron sur filtre nylon. Le hyaluronate de sodium est ajouté (16 g) sous forme de poudre dans la solution de polymère et 884 mL d'eau isotonique tamponnée est ajoutée pour ajuster les concentrations. Le mélange est secoué sur un mélangeur orbital, à une vitesse de 300 rpm, à température ambiante jusqu'à complète dissolution du hyaluronate de sodium.

Après environ 30 h de solubilisation, la solution viscoélastique aqueuse comprenant 16 mg/mL d' hyaluronate de sodium (1,6%) et 2,5 mg/mL polymère hydrosoluble de type polyéther-polyuréthane ou polyéther-polyester-uréthane (0,25%) est mise en seringue.

### Exemple 6 : conditionnement des solutions viscoélastiques aqueuses comprenant du hyaluronate de sodium et un polymère hydrosoluble de type polyéther-polyuréthane ou polyéther-polyester-uréthane

Les seringues pré-stérilisées sont remplies de 1,1 mL de solution. Elles sont ensuite stérilisées en autoclave.

L'emballage secondaire en blister de polyester est alors réalisé avant de procéder à une nouvelle stérilisation par oxyde d'éthylène (ETO).

### Exemple 7 : Solutions viscoélastiques aqueuse comprenant un polymère hydrosoluble de type polyéther-polyuréthane et du hyaluronate de sodium (NaHA)

Différentes solutions viscoélastiques aqueuses selon l'invention et leurs caractéristiques sont données dans le tableau 5 ci-dessous .

**Tableau 5**

| Polymère dans la solution | Concentration HA | Concentration polymère | Masse molaire HA | Viscosité * (Pa.s) |
|---|---|---|---|---|
| EG230 | 16 mg/mL | 2,5 mg/mL | 1,9 MDa | 130 |
| 0,25% | | | | |
| EG230 | 16 mg/mL | 1,0 mg/mL | 1,9 MDa | 110 |
| 0,1% | | | | |
| EG88(0) | 16 mg/mL | 2,5 mg/mL | 1,9 MDa | 135 |
| 0,25% | | | | |
| EG88(0) | 16 mg/mL | 1,0 mg/mL | 1,9 MDa | 110 |
| 0,1% | | | | |
| EG68(0) | 16 mg/mL | 2,5 mg/mL | 1,9 MDa | 90 |
| 0,25% | | | | |
| EG68(0) | 16 mg/mL | 1,0 mg/mL | 1,9 MDa | 100 |
| 0,1% | | | | |

| | | | | |
|---|---|---|---|---|
| * à taux de cisaillement nul déterminée par un rhéomètre TA de type AR1500EX, géométrie aluminium cône-plan, 40 mm, 63µm, 2°, T=25°C | | | | |

### Exemple 8 : Essais d'injection de lentilles intraoculaires (IOL) en présence de solutions viscoélastique aqueuse comprenant un polymère hydrosoluble de type polyéther-polyuréthane ou polyéther-polyester-uréthane

Les essais ont été réalisés avec certaines solutions viscoélastiques de l'exemple 7, en utilisant des cartouches sans aucun traitement favorisant l'éjection de la lentille (ni revêtement, ni GMS).

On procède de la manière suivante :
On introduit un implant ophtalmique dans la cartouche de chargement d'un système d'injection comprenant un corps d'injection, une cartouche de chargement (n'ayant subi aucun traitement de type coating ou GMS) et un piston d'injection.

On y ajoute une solution viscoélastique selon l'invention, à savoir une solution de NaHA ou HPMC associée à un polymère permettant le glissant dans ladite cartouche de chargement. On place le système d'injection dans un dynamomètre de type Instron 3367 équipé d'un capteur de force de sensibilité 0,5 kN, à une vitesse de compression de 8,5 mm/s. On mesure la force de compression nécessaire à l'injection de l'implant, en comparaison avec une solution viscoélastique du commerce contenant du NaHA uniquement (solution Visthesia^{®} commercialisée par Zeiss) ou une solution viscoélastique contenant HMPC uniquement.

Les résultats sont donnés dans les tableaux 6 et 7 ci-dessous.
1) Injection de lentille intraoculaire (IOL) hydrophile_au travers d'un embout Viscojet non revêtu de diamètre intérieur 2,0 mm (IOL commerciale AcriLISA^{®} de Zeiss)

**Tableau 6**

| Nature de la solution viscoélastique | Force d'injection (N) | |
|---|---|---|
| | Dioptrie 17 | Dioptrie 28 |
| NaHA 1,6%-EG88(0) 0,25% | 11 +/- 1 | 12 +/- 1 |
| NaHA 1,6%-EG88(0) 0,10% | 12 +/- 2 | 14 +/- 1 |
| NaHA 1,6%-EG230 0,25% | 13 +/- 2 | 12 +/- 0 |
| NaHA 1,6%-EG230 0,10% | 13 +/- 2 | 12 +/- 0 |
| NaHA 1,6%-EG68(0) 0,25% | - | 12 +/- 0* |
| NaHA 1,6%-EG68(0) 0,10% | - | 12 +/- 1* |
| NaHA 1,6%-ES 0,25% | 10 +/- 0 | 13 +/- 3 |
| NaHA 1,6%-ES 0,10% | 14 +/- 2 | 17 +/- 1 |
| Solution Visthesia^{®} (Zeiss) | Non éjectée | Non éjectée |
| HPMC* 1,5%- EG68(0) 0,25% | 16+/-1 | 18+/-2 |
| HPMC* 1,5% | Non éjectée | Non éjectée |

| | | |
|---|---|---|
| *HPMC de masse molaire 800KDa | | |

2) Injection de lentille intraoculaire (IOL) hydrophobe au travers d'embout Viscojet non revêtu de diamètre intérieur 2,6 mm (IOL commerciale Hydromax^{®} de Zeiss)

**Tableau 7**

| Nature de la solution viscoélastique | Force d'injection (N) | |
|---|---|---|
| | Dioptrie 11 | Dioptrie 28 |
| NaHA 1,6%-EG88(0) 0,25% | 18 +/- 4 | 27 +/- 0 |
| NaHA 1,6%-EG88(0) 0,10% | 16 +/- 1 | 28 +/- 1 |
| NaHA 1,6%-EG230 0,25% | 15 +/- 3 | 21 +/- 3 |
| NaHA 1,6%-EG230 0,10% | 14 +/- 0 | 24 +/- 0 |
| NaHA 1,6%-EG68(0) 0.25% | - | - |
| NaHA 1,6%-EG68(0) 0,10% | - | 29 +/- 0 |
| NaHA 1,6%-ES 0,25% | 20 +/- 4 | 27 +/- 4 |
| NaHA 1,6%--ES 0,10% | 24 +/- 1 | 29 +/- 2 |
| Solution Visthesia^{®} (Zeiss) | Non éjectée | Non éjectée |

Les résultats montrent que la présence de solution viscoélastique glissante (solution contenant du hyaluronate de sodium ou de l'hydroxypropylméthylcellulose associé(e) à un polymère de type polyéther-uréthane ou bien polyéther-polyester-uréthane) lors de l'injection permet à des lentilles intraoculaires, de nature hydrophobes, hydrophiles ou encore hydrophiles à surface hydrophobe, d'être injectées au travers d'embout de faible diamètre, avec des forces d'injection inférieures à 30N. Ces essais ont été réalisés avec des cartouches sans aucun traitement favorisant l'éjection de la lentille intraoculaire. Sur ce même dispositif, l'éjection d'une lentille intraoculaire, même de faible dioptrie, au moyen de solution contenant uniquement du hyaluronate de sodium ou de l'hydroxypropylméthylcellulose (sans polymère glissant) n'est pas possible.

Les valeurs de force d'injection mesurée en présence des solutions viscoélastiques utilisées selon l'invention sont comparables à celles mesurées pour des embouts d'injecteur revêtus d'un polymère glissant et utilisant une solution viscoélastique standard.

A titre de comparaison, la force nécessaire pour injecter un implant hydrophile de type AcriLISA^{®} de dioptrie 28 au travers d'une cartouche Viscojet de diamètre 2 mm revêtues par un polymère glissant est de 13 N +/- 1.

## Revendications

1. Solution viscoélastique aqueuse comprenant :
- au moins un polymère choisi parmi l'acide hyaluronique et ses sels, et un dérivé cellulosique étant l'hydroxypropylméthylcellulose ou un de ses sels, et
- au moins un polymère hydrosoluble de type polyéther-polyuréthane ou polyéther-polyester-uréthane, ledit polymère hydrosoluble de type polyéther-polyuréthane ou polyéther-polyester-uréthane ne comportant pas de chaine grasse terminale ou pendante comportant au moins 10 atomes de carbone, ladite solution viscoélastique ayant une viscosité comprise entre 10 et 7000 Pa.s.

2. Solution viscoélastique selon la revendication 1, dans laquelle l'acide hyaluronique a une masse molaire moyenne en masse comprise entre 1,5 MDa et 5 MDa.

3. Solution viscoélastique selon la revendication 1, **caractérisée en ce que** l'acide hyaluronique a une masse molaire moyenne en masse comprise entre 0,5 MDa et 1,5 MDa.

4. Solution viscoélastique selon l'une quelconque des revendications 1 à 3, dans laquelle l'acide hyaluronique est sous forme d'hyaluronate de sodium ou de potassium.

5. Solution viscoélastique selon l'une quelconque des revendications 1 à 4, dans laquelle ledit dérivé cellulosique est le sel de sodium ou le sel de potassium de l'hydroxypropylméthylcellulose.

6. Solution viscoélastique selon l'une quelconque des revendications 1 à 5, dans laquelle la teneur en polymère choisi parmi l'acide hyaluronique et ses sels, et le dérivé cellulosique ou un de ses sels, est comprise entre 0,5 et 3%, exprimée en poids/volume.

7. Solution viscoélastique selon l'une quelconque des revendications 1 à 6, dans laquelle le polymère hydrosoluble de type polyéther-polyuréthane est un copolymère à blocs d'oxyde d'éthylène et d'oxyde de propylène.

8. Solution viscoélastique selon l'une quelconque des revendications 1 à 7, dans laquelle le polymère hydrosoluble est :
- un polymère de type polyéther-polyuréthane qui est un copolymère à blocs d'oxyde d'éthylène et d'oxyde de propylène ramifié comprenant des ponts uréthane, allophanate et urée, ou
- un copolymère de type polyéther-polyester-uréthane comprenant des groupements uréthane et/ou allophanate, urée et/ou biuret.

9. Solution viscoélastique selon l'une des revendications 7 ou 8, dans laquelle ledit polymère hydrosoluble de type polyéther-polyuréthane ou de type polyéther-polyester-uréthane a une masse molaire moyenne en masse supérieure ou égale à 20 KDa, de préférence comprise entre 20 et 100 KDa.

10. Solution viscoélastique selon l'une quelconque des revendications 1 à 9, dans laquelle la teneur en polymère hydrosoluble de type polyéther-polyuréthane ou polyéther-polyester-uréthane dans la solution viscoélastique aqueuse est comprise entre 0,10 et 0,5 %, de préférence entre 0,10 et 0,25 %, exprimée en poids/volume.

11. Utilisation d'une solution viscoélastique aqueuse selon l'une quelconque des revendications 1 à 10, dans un dispositif d'injection comprenant un corps d'injection, un embout, une cartouche de chargement et un piston d'injection.

12. Utilisation d'une solution viscoélastique aqueuse selon l'une quelconque des revendications 1 à 10, dans un dispositif destiné à l'injection d'un implant intraoculaire.

13. Dispositif destiné à l'injection d'un implant intraoculaire, comprenant un corps d'injection, une cartouche de chargement et un piston d'injection, dans lequel la cartouche de chargement contient une solution viscoélastique telle que définie dans l'une quelconque des revendications 1 à 10.

14. Solution viscoélastique selon l'une quelconque des revendications 1 à 10, pour son utilisation pour l'injection d'un implant intraoculaire hydrophile ou hydrophobe.

## Patentansprüche

1. Wässrige viskoelastische Lösung, umfassend:
- mindestens ein Polymer, ausgewählt aus Hyaluronsäure und Salzen davon, und ein Zellulosederivat, das Hydroxypropylmethylcellulose oder eins der Salze davon ist, und
- mindestens ein wasserlösliches Polymer vom Typ Polyether-Polyurethan oder Polyether-Polyester-Urethan, wobei das wasserlösliche Polymer vom Typ Polyether-Polyurethan oder Polyether-Polyester-Urethan keine abschließende oder hängende Fettkette mit mindestens 10 Kohlenstoffatomen aufweist,
wobei die viskoelastische Lösung eine Viskosität zwischen 10 und 7000 Pa.s aufweist.

2. Viskoelastische Lösung nach Anspruch 1, wobei die Hyaluronsäure eine massendurchschnittliche Molmasse zwischen 1,5 MDa und 5 MDa aufweist.

3. Viskoelastische Lösung nach Anspruch 1, **dadurch gekennzeichnet, dass** die Hyaluronsäure eine massendurchschnittliche Molmasse zwischen 0,5 MDa und 1,5 MDa aufweist.

4. Viskoelastische Lösung nach einem der Ansprüche 1 bis 3, wobei die Hyaluronsäure in Form von Natrium- oder Kaliumhyaluronat vorliegt.

5. Viskoelastische Lösung nach einem der Ansprüche 1 bis 4, wobei das Zellulosederivat das Natriumsalz oder Kaliumsalz der Hydroxypropylmethylcellulose ist.

6. Viskoelastische Lösung nach einem der Ansprüche 1 bis 5, wobei der Polymergehalt, ausgewählt aus Hyaluronsäure und Salzen davon sowie Zellulosederivat oder einem seiner Salze zwischen 0,5 und 3 %, ausgedrückt in Gewicht/Volumen, beträgt.

7. Viskoelastische Lösung nach einem der Ansprüche 1 bis 6, wobei das wasserlösliche Polymer vom Typ Polyether-Polyurethan ein Blockcopolymer aus Ethylenoxid und Propylenoxid ist.

8. Viskoelastische Lösung nach einem der Ansprüche 1 bis 7, wobei das wasserlösliche Polymer ist:
- ein Polymer vom Typ Polyether-Polyurethan, das ein verzweigtes Ethylenoxid- und Propylenoxid-Blockcopolymer ist, das Urethan-, Allophanat- und Harnstoffbrücken umfasst, oder
- ein Copolymer vom Typ Polyether-Polyester-Urethan, das Urethan- und/oder Allophanat-, Harnstoff- und/oder Biuret-Gruppen umfasst.

9. Viskoelastische Lösung nach einem der Ansprüche 7 oder 8, wobei das wasserlösliche Polymer vom Typ Polyether-Polyurethan oder vom Typ Polyether-Polyester-Urethan eine massendurchschnittliche Molmasse von 20 KDa oder mehr aufweist, vorzugsweise zwischen 20 und 100 kDa.

10. Viskoelastische Lösung nach einem der Ansprüche 1 bis 9, wobei der Gehalt an wasserlöslichem Polymer vom Typ Polyether-Polyurethan oder Polyether-Polyester-Urethan in der wässrigen viskoelastischen Lösung zwischen 0,10 und 0,5 %, vorzugsweise zwischen 0,10 und 0,25 %, ausgedrückt in Gewicht/Volumen, beträgt.

11. Verwendung einer wässrigen viskoelastischen Lösung nach einem der Ansprüche 1 bis 10 in einer Injektionsvorrichtung, die einen Injektionskörper, eine Spitze, eine Ladekartusche und einen Injektionskolben umfasst.

12. Verwendung einer wässrigen viskoelastischen Lösung nach einem der Ansprüche 1 bis 10 in einer Vorrichtung, die zur Injektion eines intraokularen Implantats bestimmt ist.

13. Vorrichtung, die zur Injektion eines intraokularen Implantats bestimmt ist, umfassend einen Injektionskörper, eine Ladekartusche und einen Injektionskolben, wobei die Ladekartusche eine viskoelastische Lösung nach einem der Ansprüche 1 bis 10 enthält.

14. Viskoelastische Lösung nach einem der Ansprüche 1 bis 10 zu ihrer Verwendung zur Injektion eines hydrophilen oder hydrophoben intraokularen Implantats.

## Claims

1. Aqueous viscoelastic solution comprising:
- at least one polymer selected from hyaluronic acid and salts thereof, and a cellulosic derivative being hydroxypropyl methylcellulose and salts thereof, and
- at least one water-soluble polymer of the polyether polyurethane or polyether polyester urethane type, said water-soluble polymer of polyether polyurethane or polyether polyester urethane type not having terminal or pendant fatty acid chain comprising at least 10 carbon atoms,
said viscoelastic solution having a viscosity comprised between 10 and 7000 Pa.s.

2. Viscoelastic solution according to claim 1, in which the hyaluronic acid has a weight average molecular weight comprised between 1.5 MDa and 5 MDa.

3. Viscoelastic solution according to claim 1, **characterized in that** the hyaluronic acid has a weight average molecular weight comprised between 0.5 MDa and 1.5 MDa.

4. Viscoelastic solution according to any one of claims 1 to 3, in which the hyaluronic acid is in the form of sodium or potassium hyaluronate.

5. Viscoelastic solution according to any one of claims 1 to 4, in which said cellulosic derivative is the sodium salt or potassium salt of hydroxypropyl methylcellulose.

6. Viscoelastic solution according to any one of claims 1 to 5, in which the content of polymer selected from hyaluronic acid and salts thereof, and the cellulosic derivative or one of the salts thereof, is comprised between 0.5 and 3%, expressed in weight/volume.

7. Viscoelastic solution according to any one of claims 1 to 6, in which the water-soluble polymer of the polyether polyurethane type is an ethylene oxide and propylene oxide block copolymer.

8. Viscoelastic solution according to any one of claims 1 to 7, in which the water-soluble polymer is:
- a polymer of the polyether polyurethane type that is a branched ethylene oxide and propylene oxide block copolymer comprising urethane, allophanate and urea bridges, or
- a copolymer of the polyether polyester urethane type comprising urethane, and/or allophanate, urea and/or biuret groups.

9. Viscoelastic solution according to one of claims 7 or 8, in which said water-soluble polymer of the polyether polyurethane type or of polyether polyester urethane type has a weight average molecular weight greater than or equal to 20 KDa, preferably comprised between 20 and 100 KDa.

10. Viscoelastic solution according to any one of claims 1 to 9, in which the content of water-soluble polymer of the polyether polyurethane or polyether polyester urethane type in the aqueous viscoelastic solution is comprised between 0.10 and 0.5%, preferably between 0.10 and 0.25%, expressed as weight/volume.

11. Use of an aqueous viscoelastic solution according to any one of claims 1 to 10, in an injection device comprising an injection body, a tip, a loading cartridge and an injection piston.

12. Use of an aqueous viscoelastic solution according to any one of claims 1 to 10, in a device intended for the injection of an intraocular implant.

13. Device intended for the injection of an intraocular implant, comprising an injection body, a loading cartridge and an injection piston, in which the loading cartridge contains a viscoelastic solution as defined in any one of claims 1 to 10.

14. Viscoelastic solution according to any one of claims 1 to 10, for use thereof for the injection of a hydrophilic or hydrophobic intraocular implant.
